# EUROPEAN PATENT SPECIFICATION

(11) **EP 2 603 503 B1**
(45) Date of publication and mention of the grant of the patent: **05.08.2015**
(21) Application number: 11764455.9
(22) Date of filing: 27.09.2011
(51) Int. Cl.: C07D 403/12, A61K 31/4439, A61P 9/00

(54) **DABIGATRAN ETEXILATE BISMESYLATE SALT, SOLID STATE FORMS AND PROCESS FOR PREPARATION THEREOF**
DABIGATRAN-ETEXILAT-BISMESYLATSALZ, FESTE FORMEN UND VERFAHREN ZU IHRER HERSTELLUNG
MÉSYLATE DE DABIGATRAN ÉTEXILATE, SES FORMES SOLIDES ET LEURS PROCÉDÉS DE PRÉPARATION

(30) Priority: 27.09.2010 EP 10179845; 24.11.2010 US 417022 P
(43) Date of publication of application: 19.06.2013
(73) Proprietor: ratiopharm GmbH, 89079 Ulm (DE)
(72) Inventor: FISCHER, Dirk, 89073 Ulm (DE)
(74) Representative: Gallagher, Kirk James
(86) International application number: PCT/US2011/053381
(87) International publication number: WO 2012/044595

(56) References cited:
- WO-A1-03/074056
- WO-A1-2005/028468

## Description

### Field of the Invention

The present invention relates to Dabigatran etexilate bismesylate, a crystalline form thereof, and a pharmaceutical composition comprising it, or said crystalline form thereof; and methods of preparing the Dabigatran etexilate bismesylate, said crystalline form thereof and said pharmaceutical composition.

### Background of the Invention

Dabigatran etexilate mesylate has the chemical structure:

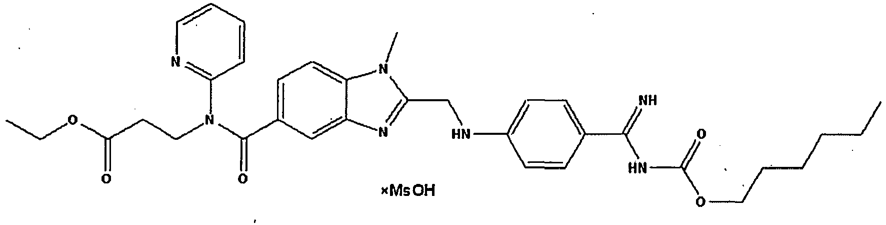

Dabigatran etexilate mesylate has the chemical name, ethyl 3-{[(2-{[(4-{N'-[(hexyloxy)carbonyl]carbamimidoyl}phenyl)amino]methyl}-1-methyl-1H-benzimid-azol-5-yl)carbonyl] (2-pyridinyl)amino}propanoate mesylate salt. Dabigatran etexilate mesylate is an anticoagulant drug from the class of the direct thrombin inhibitors. Dabigatran etexilate mesylate is marketed under the trade name PRADAXA® by Boehringer Ingelheim.

WO 2003/074056, WO 2005/028468 and WO 2005/023249 disclose a mesylate salt of Dabigatran etexilate, as well as crystalline forms, which is, in all those publications, a monomesylate salt.

WO 2005/028468 discloses crystalline forms of Dabigatran etexilate mesylate. The salt is suggested, according to the WO 2005/028468 publication, to increase the solubility of Dabigatran etexilate.

WO 2006/114415 and WO 2008/043759 disclose additional salts of Dabigatran etexilate.

Polymorphism, the occurrence of different crystal forms, is a property of some molecules and molecular complexes. A single molecule, like Dabigatran etexilate mesylate, may give rise to a variety of polymorphic forms having distinct crystal structures and physical properties like melting point, powder X-ray diffraction (PXRD) pattern, infrared absorption fingerprint, and solid state NMR spectrum. One polymorphic form may give rise to thermal behavior different from that of another polymorphic form. Thermal behavior can be measured in the laboratory by such techniques as capillary melting point, thermogravimetric analysis (TGA), and differential scanning calorimetry (DSC) as well as content of solvent in the polymorphic form, which have been used to distinguish polymorphic forms.

The difference in the physical properties of different salts and polymorphic forms results from the orientation and intermolecular interactions of adjacent molecules or complexes in the bulk solid. Accordingly, polymorphs are distinct solids sharing the same molecular formula yet having distinct advantageous physical properties compared to other polymorphic forms of the same compound or complex.

One of the most important physical properties of pharmaceutical compounds is their solubility in aqueous solution, particularly their solubility in the gastric juices of a patient. For example, where absorption through the gastrointestinal tract is slow, it is often desirable for a drug that is unstable to conditions in the patient's stomach or intestine to dissolve slowly so that it does not accumulate in a deleterious environment

The discovery of new salts of Dabigatran etexilate and and polymorphic forms thereof may provide new opportunities to improve the synthesis and the characteristics of the active pharmaceutical ingredient (API). Such discoveries can also enlarge the repertoire of materials that a formulation scientist has available for designing, for example, a pharmaceutical dosage form of a drug with a targeted release profile or other desired characteristic.

### Summary of the Invention

The present invention provides a bismesylate salt of Dabigatran etexilate.

The present invention further provides the bismesylate salt of Dabigatran etexilate and its crystalline form for use in the preparation of Dabigatran etexilate and Dabigatran etexilate salts, such as the monomesylate.

The present invention further provides a process for preparing Dabigatran etexilate and salts thereof, such as the monomesylate salt, comprising preparing the bismesylate salt of Dabigatran etexilate according to the present invention, and converting it to Dabigatran etexilate or a salt thereof.

The present invention also provides a pharmaceutical composition comprising Dabigatran etexilate bismesylate salt. The present invention further provides a pharmaceutical composition comprising Dabigatran etexilate bismesylate salt for use in preventing blood clots in a patient suffering from, or susceptible to, the formation of blood clots.

### Brief Description of the Drawings

Figure 1 shows an X-ray powder diffractogram ("PXRD") pattern of crystalline Dabigatran etexilate bismesylate form, which may be designated form Ms2-A.
Figure 2 shows an X-ray powder diffractogram ("PXRD") pattern of crystalline Dabigatran etexilate bismesylate form, which may be designated form Ms2-A (prepared according to example 2).
Figure 3 shows DSC thermogram of crystalline Dabigatran etexilate bismesylate form Ms2-A
Figure 4 provides a solid state ¹³C NMR pattern of crystalline Dabigatran etexilate bismesylate form Ms2-A
Figure 5 provides a solid state ¹³C NMR pattern of crystalline Dabigatran etexilate bismesylate form Ms2-A

### Detailed Description of the Invention

The present invention relates to Dabigatran etexilate bismesylate, its crystalline form, and a pharmaceutical composition comprising it; as well as a method of preparing the same.

A crystal form may be referred to herein as being characterized by graphical data substantially "as depicted in" a Figure. Such data include, for example, powder X-ray diffractograms, FTIR spectra and solid state NMR spectra. The skilled person will understand that such graphical representations of data may be subject to small variations, e.g., in peak relative intensities and peak positions due to factors such as variations in instrument response and variations in sample concentration and purity, which are well known to the skilled person. Nonetheless, the skilled person would readily be capable of comparing the graphical data in the Figures herein with graph-ical data generated for an unknown crystal form and confirm whether the two sets of graphical data are characterizing the same crystal form or two different crystal forms.

A crystal form (or polymorph) may be referred to herein as polymorphically pure, or substantially free of any other crystalline (or polymorphic) forms of Dabigatran etexilate bismesylate or of Dabigatran etexilate monomesylate. As used herein in this context, the expression "substantially free'' will be understood to mean that the crystalline form contains 20% or less, 10% or less, 5% or less, 2% or less, or 1% or less of any other form of the subject compound as measured, for example, by PXRD. Thus, polymorphs of Dabigatran etexilate bismesylate described herein as substantially free of any other polymorphic forms would be understood to contain greater than 80% (w/w), greater than 90% (w/w), greater than 95% (w/w), greater than 98% (w/w), or greater than 99% (w/w) of the subject polymorphic form of Dabigatran etexilate monomesylate or Dabigatran etexilate bismesylate . Accordingly, in some embodiments of the invention, the described polymorphs of Dabigatran etexilate bismesylate may contain from 1% to 20% (w/w), from 5% to 20% (w/w), from 5% to 10%, from 5% to 1% (w/w) or from 2% to 1% (w/w), preferably 1% (w/w) or less of one or more other crystal forms of Dabigatran etexilate monomesylate or Dabigatran etexilate bismesylate.

The terms "XRPD" and "PXRD" are used interchangeably in the scientific literature by those skilled in the art of powder X-ray diffraction analysis, and the present application makes no distinction between these two expressions or their abbreviations.

As used herein, unless stated otherwise, the PXRD measurements are taken using copper Kα radiation wavelength 1.5406A.

As used herein, the term "wet crystalline form" refers to a polymorph that was not dried using any conventional techniques to remove residual solvent.

As used herein, the term "dry crystalline form" refers to a polymorph that was dried using any conventional techniques to remove residual solvent.

A thing, *e.g.,* a reaction mixture, may be characterized herein as being at, or allowed to come to "room temperature", often abbreviated "RT." This means that the temperature of the thing is close to, or the same as, that of the space, *e.g.,* the room or fume hood, in which the thing is located. Typically, room temperature is from about 15°C to about 30°C, or about 20°C to about 25°C, or about 25°C.

A process or step may be referred to herein as being carried out "overnight." This refers to a time interval, *e.g.,* for the process or step, that spans the time during the night, when that process or step may not be actively observed. This time interval is from about 8 to about 20 hours, or about 10-18 hours, typically about 16 hours.

As used herein, unless indicated otherwise, the term "isolated" in reference to Dabigatran etexilate bismesylate or its crystalline form corresponds to Dabigatran etexilate bismesylate (or its crystalline form) that is physically separated from the reaction mixture, where it is formed.

As used herein, the term "absolute ethanol" refers to ethanol having 1% (weight/ weight percentage) or less of water, or 0.5% or less of water, particularly, 0.25% or less of water, more particularly, 0.15% or less of water.

As used herein, and unless stated otherwise, the term "anhydrous" in relation to Dabigatran etexilate bismesylate and/or its crystalline form relates to a crystalline Dabigatran etexilate bismesylate which contains not more than 1% (w/w), more preferably not more than 0.5% (w/w) of either water or organic solvents incorporated in the crystalline structure, as measured by TGA.

The term "solvate," as used herein and unless indicated otherwise, refers to a crystal form that incorporates a solvent in the crystal structure. When the solvent is water, the solvate is often referred to as a "hydrate." The solvent in a solvate may be present in either a stoichiometric or in a non-stoichiometric amount.

The present invention provides Dabigatran etexilate bismesylate salt and its crystalline form. The salt and the crystalline form of the present invention may have advantageous properties selected from at least one of: chemical purity, flowability, solubility, morphology or crystal habit, stability - such as storage stability, stability to dehydration, stability to polymorphic conversion, low hygroscopicity, low content of residual solvents. In particular, the Dabigatran etexilate bismesylate salt and its crystalline form, described in the present invention, has an improved solubility, particularly in comparison to the monomesylate salt; and it is stable upon storage at a temperature of about 25°C and relative humidity (RH) of about 60%, or upon storage temperature of about 40°C and RH of about 75%; for a period of at least two weeks.

Dabigatran etexilate has solubility in water of only 1.8 mg/ml. Moreover, the solubility strongly depends on the pH. In acid environment the solubility is strongly increased. This results in problems in the bioavailability of the active ingredient because its solubility depends on the pH value in the stomach of the patient. Particular problems arise for patients having a stomach wherein the pH value is altered, for example due to physiolgical variabiliy, diseases or promedication, such as due to proton pump (PP) inhibitors.

It has now surprisingly been found that the above and further problems are solved by providing a salt of Dabigatran etexilate with methanesulfonic acid in a molar ratio of about 1:2. Therefore, the present invention relates to Dabigatran etexilate bismesylate. The solubility of the bismesylate salt of Dabigatran etexilate is significantly increased compared to the solubility of the known monomesylate salt. While Dabigatran etexilate monomesylate has solubility in water at 37 °C of 14.7 mg/ml the Dabigatran etexilate bismesylate of the present invention has solubility in water at 37 °C of 33.9 mg/ml. Moreover, the high solubility of Dabigatran etexilate bismesylate results in a high bioavailability of the active ingredient.

In one embodiment the invention encompasses Dabigatran etexilate bismesylate salt.

The above Dabigatran etexilate bismesylate of the present invention can be amorphous or crystalline. Moreover, it can be in the form of a hydrate or solvate. Crystalline forms of Dabigatran etexilate bismesylate include different polymorphic crystalline forms. Furthermore, the Dabigatran etexilate bismesylate of the present invention can be in the form of a mixture of amorphous and crystalline fractions.

Preferably, the above Dabigatran etexilate bismesylate salt is isolated, preferably in a crystalline form.

Preferably, the above Dabigatran etexilate bismesylate salt is isolated, preferably in a crystalline form.

In a preferred embodiment the present invention provides Dabigatran etexilate bismesylate in a certain polymorphic crystalline form which shows an XRPD pattern having peaks at 15.8 ± 0.2 ° 2-Theta, 20.4 ± 0.2° 2-Theta and 24.3 ± 0.2 ° 2-Theta, preferably the Dabigatran etexilate bismesylate has one two or three additional peaks selected from 19.1 ± 0.2 ° 2-Theta, 23.6 ± 0.2 ° 2-Theta, and 25.8 ± 0.2 ° 2-Theta.

In a more preferred embodiment the present invention provides Dabigatran etexilate bismesylate in a certain polymorphic crystalline form which shows an XRPD pattern having peaks at 15.8 ± 0.2 ° 2-Theta, 19.1 ± 0.2 ° 2-Theta, 20.4 ± 0.2 °2-Theta, 23.6 ± 0.2° 2-Theta, 24.3 ± 0.2 ° 2-Theta and 25.8 ± 0.2 ° 2-Theta.

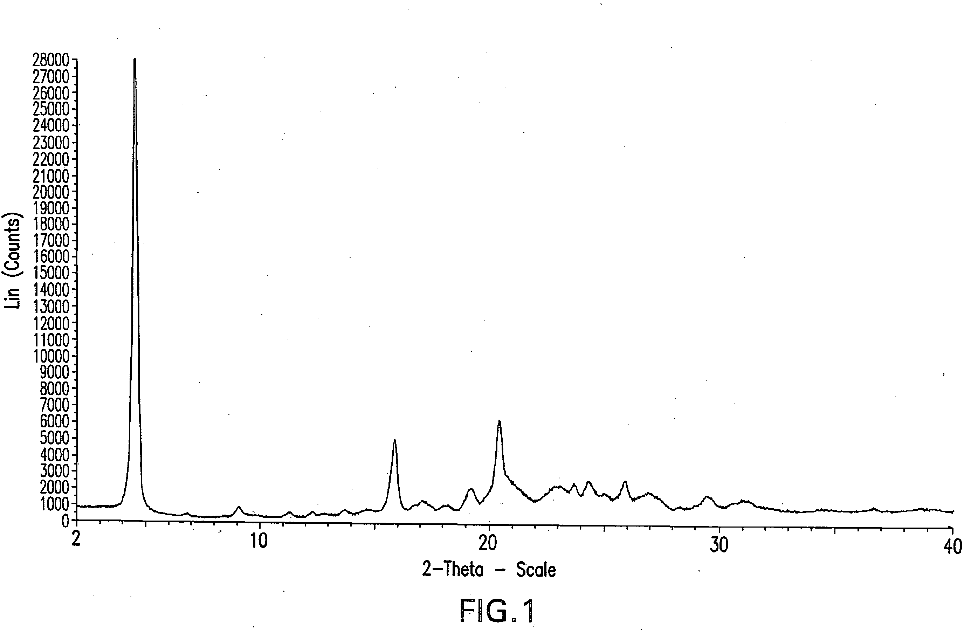

In a particularly preferred embodiment the polymorphic crystalline form of dabigatran etexilate bismesylate of the present invention shows substantially the XRPD pattern of Figure 1. The main peaks of this pattern are summarized in the following Table 1.

| **Angle 2-Theta ° ± 0.2** | **Intensity %** |
|---|---|
| 12.2 | 21 |
| 15.8 | 76 |
| 17.0 | 36 |
| 19.1 | 72 |
| 19.8 | 52 |
| 20.4 | 100 |
| 20.7 | 75 |
| 23.6 | 69 |
| 24.3 | 80 |
| 25.0 | 74 |
| 25.8 | 52 |
| 29.4 | 44 |

This crystalline form can be designated as form Ms2-A.

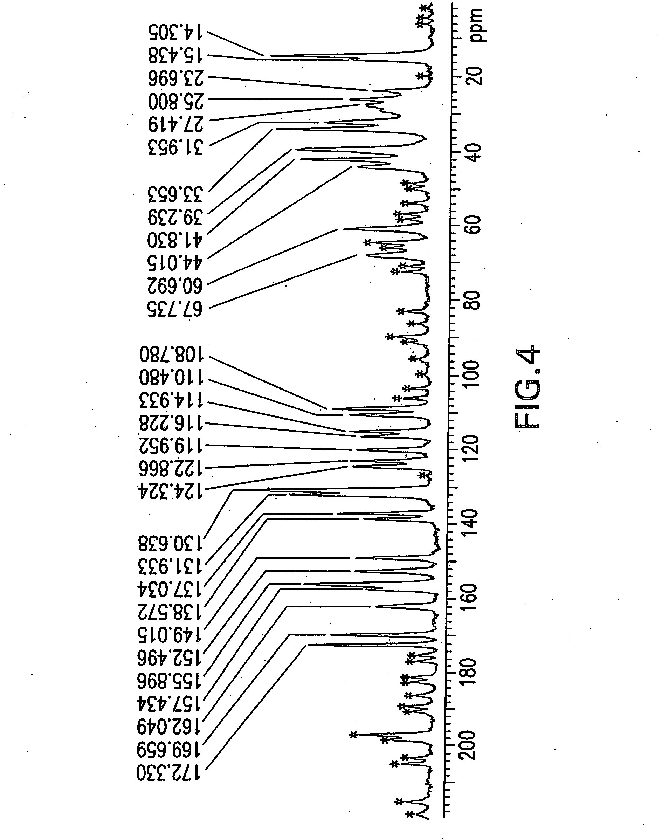

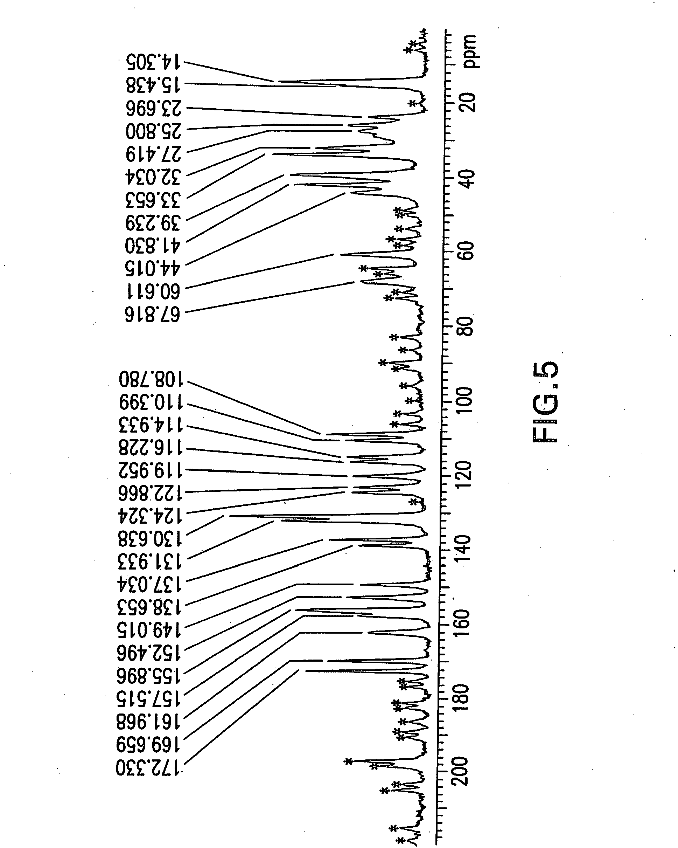

Form Ms2-A can also be characterized by a solid-state ¹³C NMR spectrum having characteristic peaks at 33.7,41.8, 114.9, 120.0,130.6 and 149.0 ppm, ± 0.2; a solid-state ¹³C NMR spectrum substantially as depicted in figure 4 or in figure 5; or by combinations thereof.

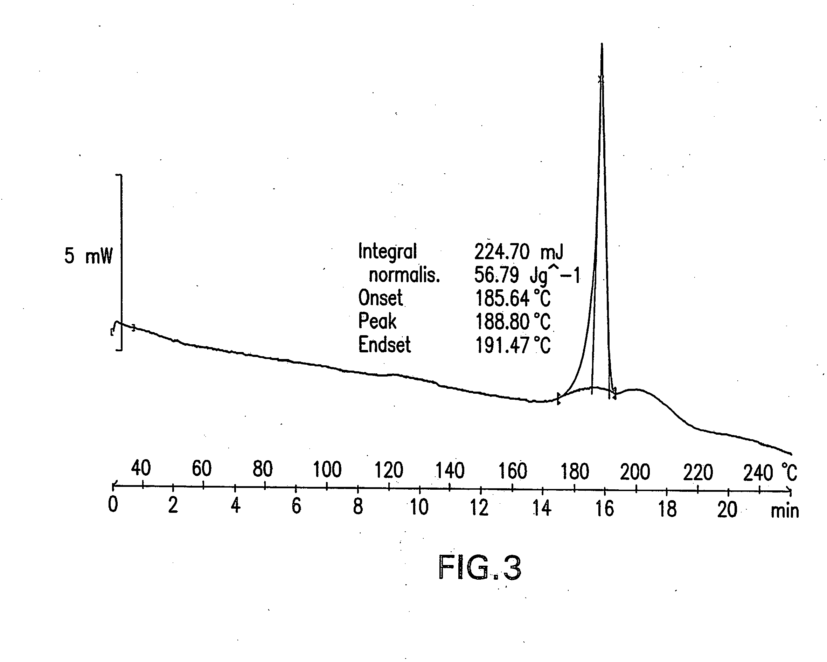

In a particularly preferred embodiment the form Ms2-A of Dabigatran etexilate bismesylate of the present invention can be characterized by a DSC thermogram substantially as depicted in Figure 3. In addition, form Ms2-A can be characterized by any combination of the above data.

The above described crystalline form of Dabigatran etexilate bismesylate can be an anhydrous form.

The above described Dabigatran etexilate bismesylate can be prepared by a process comprising dissolving dabigatran etexilate, preferably dabigatran etexilate free base, or Dabigatran etexilate monomesylate, in a solvent; and contacting the obtained solution with methanesulfonic acid to form a mixture, whereby the molar ratio of Dabigatran etexilate to methanesulfonic acid is of about 1: 1.8 or lower, preferably between about 1: 1.8 and 1: 2.2, preferably 1: 2, and most preferably about 1:1.9.

Any solvent suitable for dissolving Dabigatran etexilate can be used in the above method. Suitable solvents include, for example, organic solvents, such as acetone and ethyl acetate.

The methanesulfonic acid can be added to the solution of Dabigatran etexilate in undiluted or diluted form. If diluted, the methanesulfonic acid can be mixed with the same or a different solvent as used in the preparation of the solution of the dabigatran etexilate. In a preferred embodiment the methanesulfonic acid is added as a solution in the same solvent as used in the preparation of the Dabigatran etexilate solution.

The methanesulfonic acid can be added to the Dabigatran etexilate solution immediately in one portion or over a prolonged period of time, such as dropwise. For example, a diluted methanesulfonic acid solution can be added to the Dabigatran etexilate solution over a period of 15 - 40 minutes. The resulting mixture may then be maintained, optionally with stirring, for a suitable time, during which time precipitation of the product occurs.

The formation of the Dabigatran etexilate bismesylate can be carried out at any suitable temperature, such as room temperature or any other suitable temperature, a suitable solvent, such as acetone, and dried, for example at elevated temperature of for example 50 °C in vacuum.

The present invention also provides a process for preparing Dabigatran etexilate bismesylate in a high degree of crystallinity. It was surprisingly found that the crystallinity of the Dabigatran etexilate bismesylate obtained by the above described or any other method can be increased by contacting the Dabigatran etexilate bismesylate with acetone at an elevated temperature. The increase in crystallinity can be observed, for example, by comparing the XRPD patterns of the Dabigatran etexilate bismesylate before and after the treatment with warm acetone. In a preferred embodiment, the present invention provides a method of increasing the crystallinity of Dabigatran etexilate bismesylate comprising contacting Dabigatran etexilate bismesylate with acetone at an elevated temperature. Preferably the temperature is in the range from 40 °C to the boiling point of the mixture, preferably the temperature is in the range from about 40 °C to about 70 °C, and more preferably about 50 to about 65 °C.. The contact time is not particularly limited and can be for example four hours or more, such as about six hours.

The Dabigatran etexilate bismesylate salt and its crystalline form described above can be used to prepare Dabigatran etexilate and Dabigatran etexilate salts, such as the monomesylate, as well as pharmaceutical compositions thereof.

In one embodiment the invention encompasses a process for preparing Dabigatran etexilate monomesylate comprising preparing bismesylate salt of Dabigatran etexilate and/or its crystalline form according to the present invention and converting it to Dabigatran etexilate monomesylate. The conversion can be done either by first converting the bismesylate salt to the free base form and reacting with sufficient amount of methanesulfonic acid, or, alternatively, the conversion can be done by basifying the bismesylate salt directly to the monomesylate form. The process can be performed, for example, by basifying the bismesylate salt of Dabigatran etexilate, preferably a solution of the said salt, and, if necessary, reacting with methanesulfonic acid in an amount suitable to obtain a monomesylate salt. Such amount, typically, can be at a molar ratio of Dabigatran etexilate to methanesulfonic acid from about 1: 0.95 to about 1: 0.99, preferably between about 1: 0.95 to 1: 0.98.

The Dabigatran etexilate bismesylate salt and its crystalline form described above can also be used to prepare pharmaceutical compositions.

The present invention provides the bismesylate salt of Dabigatran etexilate and its crystalline form for use in the preparation of pharmaceutical composition.

The present invention also provides a pharmaceutical composition comprising Dabigatran etexilate and its crystalline form. This pharmaceutical composition may additionally comprise at least one pharmaceutically acceptable excipient.

The present invention also provides the use of the above described Dabigatran etexilate and its crystalline form for the manufacture of a medicament.

The present invention further provides a pharmaceutical compostion comprising Dabigatran etexilate bismesylate for use in preventing blood clots in a patient suffering from, or susceptible to, the formation of blood clots.

The present invention furthermore relates to a pharmaceutical preparation comprising Dabigatran etexilate bismesylate as active pharmaceutical ingredient. The pharmaceutical preparation of the present invention preferably is an oral solid preparation, such as a capsule or tablet.

The pharmaceutical preparation can additionally contain one or more pharmaceutically acceptable excipients, such as fillers, glidants, disintegrants, flow regulating agents and release agents. Suitable excipients are for example disclosed in "Lexikon der Hilfsstoffe für Pharmazie, Kosmetik und angrenzende Gebiete", published by H.P. Fielder, 4th Edition and "Handbook of Pharmaceutical Excipients", 3rd Edition, published by A.H. Kibbe, American Pharmaceutical Association, Washington, USA, and Pharmaceutical Press, London.

Suitable fillers are for example lactose and calcium hydrogen phosphate. Fillers can be present in an amount of 0 - 80% by weight, preferably in an amount of 10 - 60% by weight of the total weight of the composition.

Suitable glidants are for example alkaline earth metal salts of fatty acids, like stearic acid. The glidant can be present for example in an amount of 0 - 2% by weight, preferably in an amount of 0.5 -1.5% by weight of the total weight of the composition.

Suitable disintegrants are for example crosscarmelose sodium, sodium carboxymethyl starch, crosslinked polyvinylpyrrolidone (crosspovidone), sodium carboxymethylglycolate (such as Explotab) and sodium bicarbonate. The disintegrant can be present in an amount of 0 - 20% by weight, preferably in an amount of 1 - 15% by weight of the total weight of the composition.

A suitable flow regulating agent is for example colloidal silica. The flow regulating agent can be present in an amount of 0 - 8% by weight, preferably in an amount of 0.1 - 3% by weight of the total weight of this composition.

A suitable release agent is for example talcum. The release agent can be present in an amount of 0 - 5% by weight, preferably in an amount of 0.5 - 3% by weight of the total weight of the composition.

The pharmaceutical preparation of the present invention can be prepared by methods well known to a person skilled in the art.

Having described the invention with reference to certain preferred embodiments, other embodiments will become apparent to one skilled in the art from consideration of the specification. The invention is further defined by reference to the following examples describing in detail the preparation of the composition and methods of use of the invention. It will be apparent to those skilled in the art that many modifications, both to materials and methods, may be practiced without departing from the scope of the invention.

### X-ray powder diffractogram ("PXRD") method:

The PXRD samples were analysed on a Bruker-AXS D8 Advance powder X-Ray diffractometer. The measurement conditions were as follows:
Measurement in Bragg-Brentano-Geometry on vertical goniometer (reflection, theta/theta, 435 mm measurement circle diameter) with sample rotation (30 rpm) on 9 position sample stage

| | |
|---|---|
| Radiation: | Cu Kα1(1.5406Å), Tube (Siemens FLCu2K), power 40kV/40mA |
| Detector: | position sensitive detector VANTEC-1, 3° capture angle (2theta) |
| Anti scatter slit: | 6.17 mm |
| Detector slit: | 10.39 mm, 4° soller slit, primary beam stop (<2° 2theta) |
| Monochromator: | None |
| Second β filter: | Ni filter 0.1 mm (0.5%) |
| Start Angle: | 2° |
| End Angle: | 55° |
| Step: | 0.017° 2Theta |
| Step Time: | 70 sec. |
| Software: | EVA (Bruker-AXS, Karlsruhe). |

### Solid state ¹³C-NMR method:

NMR spectra of solid samples were recorded on Varian NMR System 600 MHz NMR spectrometer equipped with 3.2 mm NB Double Resonance HX MAS Solids Probe. Larmor frequency of carbon nuclei was 150.79 MHz. The ¹³C CP-MAS NMR spectra were externally referenced methyl group of hexamethylbenzene (HMB) (δ=17.3 ppm). Samples were spun at the magic angle with 10 kHz during ¹³C measurement.
¹³C: The pulse sequence used for acquiring the spectrum was a standard cross-polarization MAS pulse sequence with high-power proton decoupling during acquisition. Repetition delay was 5 s and the number of scans was 400.

### GC-MS method

### Instrument:

| | |
|---|---|
| Headspace sampler | |
| instrument: | Agilent G 1888 |
| oven: | 70 °C |
| loop: | 100 °C |
| transfer line: | 120°C |
| vial equilibration: | 15 min |
| pressure: | 0.2 min |
| loop fill: | 0.2 min |
| inject time: | 0.5 min |

### GC-MS:

### instruments

| | | |
|---|---|---|
| GC: | Agilent 7890A | |
| MSD: | Agilent 5975C | |
| method: | HS EP 35.M | |
| column: | Agilent DB-624 30m x 250 µm x 1.4 µm | |
| temperature program: | 35 °C, 5 min isothermal | |
| | 35-190°C, 5°C / min | |
| | 190°C, 9.4 min isothermal | |
| run time : | 45.4 min | |
| carrier gas : | helium | flow :12.9 ml / min |
| pressure: | 12.4 psi | |
| injection: | split | split ratio : 10 :1 |
| temperature : | inlet : | 220 °C |
| | GC-MS interface: | 280 °C |
| | MS source : | 230°C |
| | MS quad. : | 150 °C |
| MSD pressure: | 9.8 x 10⁻⁶ Torr | |
| tune file : | atune.u | |
| emission: | 34.6 µA | |
| electron energy : | 69.9 eV | |
| repeller: | 27.8 V | |
| ion focus : | 90.2 V | |
| entrance lence : | 25.5 V | |
| EM volts: | 1129.4 V | |
| solvent delay: | 2.5 min | |
| scan : | 35 -500 m/z | |

### Sample Preparation

Into the headspace vial, 10 mg of the sample were dissolved in 1 ml DMSO and this solution was directly subjected to Headspace-GC-MS analysis.

A calibration solution, containing 50 µg acetone, was prepared by adding 63 µl acetone to 10 ml DMSO (conc.= 500 µg/ml), 100 µl of this solution were diluted with 900 µl DMSO and 100 µl of this solution were diluted with 900 µl DMSO (conc.= 50 ¡..t.g/ml, equivalent to 5,000 ppm based on a sample weight of 10 mg).

A calibration solution, containing 50 µg ethyl acetate, was prepared by adding 55 µl ethyl acetate to 10 ml DMSO (conc.= 500 µg/ml) 100 µl of this solution were diluted with 900 µl DMSO and 100 µl of this solution were diluted with 900 µl DMSO (conc.= 50 µg/ml, equivalent to 5,000 ppm based on a sample weight of 10 mg).

### Differential Scanning Calorimetry method

| | |
|---|---|
| Apparatus: | Mettler Toledo DSC 822E coupled with a Mettler Toledo Gas-Flow-Controller TS0800GC1 (Mettler-Toledo GmbH, Giessen, Germany) |
| Aluminium crucible: | 40 µL |
| Lid: | perforated |
| Temperature range: | 30 °C to 300 °C |
| Heating rate: | 10°C/ min |
| Nitrogen flush: | 50 mL / min |
| Software: | STARe Version. 8.10 |
| Interpretation: | Endothermic modus |

The invention will now be illustrated by the examples which are not construed as being limiting.

### Examples

### Reference examples:

### Preparation of starting material: Dabigatran etexilate mesylate form I according to US 2005/0234104 example 1:

Ethyl 3-[(2-{[4-(hexyloxycarbonylaminoiminomethyl)phenylamino]methyl}-1-methyl-1H-benzimidazole-5-carbonyl)pyridin-2-ylamino]propionate base (52.6 kg) (which has preferably been purified beforehand by recrystallization from ethyl acetate) is placed in an agitator apparatus which has been rendered inert and then 293 kg of acetone is added. The contents of the apparatus are heated to 40° C to 46° C with stirring. After a clear solution has formed, the contents of the apparatus is filtered into a second agitator apparatus through a lens filter and then cooled to 30° C to 36° C. 33 kg of acetone precooled to 0° C to 5° C, 7.9 kg of 99.5% methanesulfonic acid, and for rinsing another 9 kg of acetone are placed in the suspended container of the second apparatus. The contents of the suspended container are added in metered amounts to the solution of ethyl 3-[(2-{[4-(hexyloxycarbonylamino-iminomethyl)phenylamino]methyl}-1-methyl-1H-benzimidazole-5-carbonyl)pyridin-2-ylamino]propionate base at 26° C to 36° C within 15 to 40 minutes. Then the mixture is stirred for 40 to 60 minutes at 26° C to 33° C. It is then cooled to 17° C to 23° C and stirred for a further 40 to 80 minutes. The crystal suspension is filtered through a filter dryer and washed with a total of 270 L of acetone. The product is dried in vacuum at a maximum of 50° C for at least 4 hours. Yield: 54.5-59.4 kg; 90%-98% of theory based on ethyl 3-[(2-{[4-(hexyloxycarbonyl-aminoiminomethyl)phenylamino]methyl}-1-methyl-1H-benzimidazole-5-carbonyl)-pyridin-2-ylamino]propionate base.

### Preparation of starting material: Dabigatran Etexilate free base

Dabigatran Etexilate free base can be prepared according to the procedures disclosed in US 6087380 - example 113 or US 7202368 - example 5

### Example 1

2.08 g of dabigatran etexilate free base was dissolved in 14.7 ml of acetone at 30 - 36 °C. 0.210 ml of methanesulfonic acid diluted in 2.20 ml of acetone was added within 15 - 40 min. at 26 - 36 °C. The resulting mixture was first steered for 40 - 60 min. at 26 - 36 °C and then for 40 - 80 min at 17 - 23 °C.

The resulting crystalline product was filtered off, washed with 17.87 ml of acetone and dried at 50 °C for 18 hours at 540 mbar.

### Example 2

1.0 g of dabigatran etexilate free base was suspended in 80.0 ml of ethylacetate. 0.2 ml of methanesulfonic acid diluted in 7.21 ml of ethylacetate was added at room temperature. The precipitated product was filtered off.

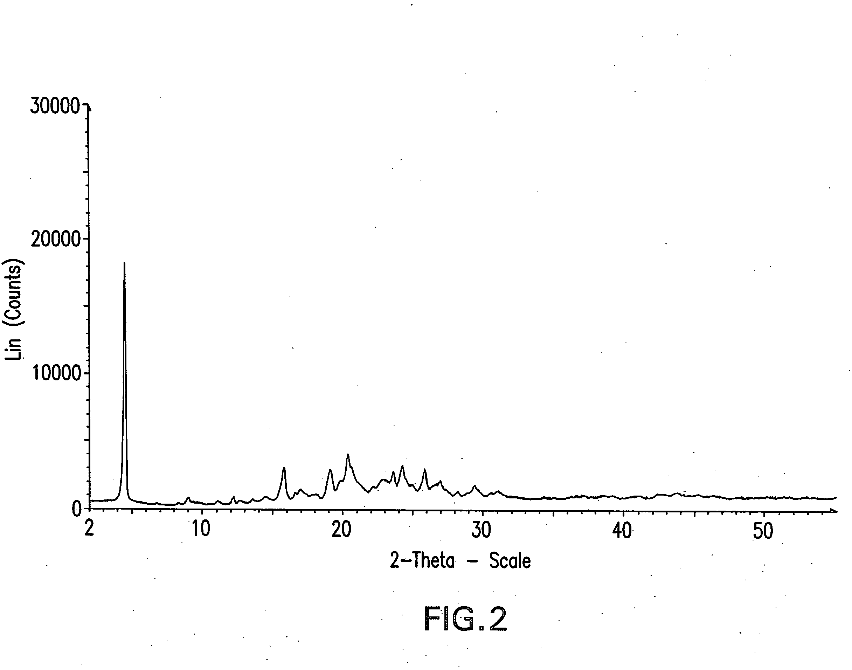

The XRPD pattern of the resulting product is shown in Figure 2.

### Example 3

Part of the product of example 2 was steered in acetone at 60 °C for six hours. The XRPD pattern of the resulting product is shown in Figure 1.

### Example 4

Part of the product of example 2 was steered in acetone at 60 °C for six hours. The obtained suspension was filtered, washed with acetone and dried, The XRPD pattern of the resulting product is shown in Figure 1.

### Example 5

In a 1L 3-neck round bottom flask, 500 ml ethyl acetate and 10 g (13.7 mmol) Dabigatran etexilate mesylate (Form I) were stirred at ambient temperature. 1.29 g (13.5 mmol) methanesulfonic acid dissolved in 62 ml ethyl acetate were added dropwise and the mixture was stirred overnight. The obtained precipitate was filtered off, washed with 20 ml acetone and dried at 40°C in the vacuum (15 mbar) overnight. The product was analyzed by PXRD and found to be Dabigatran etexilate bismesylate form Ms2-A.
Yield: 11.25 g (99.9%)
Chemical purity (HPLC): 99.67 %
DSC: peak of endotherm at 188.8°C
Residual solvent content (ethyl acetate): Below limit of detection

### Example 6

In a 500 ml L 3-neck round bottom flask, 200 ml acetone and 10 g (13.7 mmol) DAB etexilate mesylate (Form I) were stirred at ambient temperature. 1.29 g (13.5 mmol) methanesulfonic acid dissolved in 50 ml ethyl acetate were added dropwise and the mixture was stirred overnight. The obtained precipitate was filtered off, washed with 30 ml acetone and dried at 40°C in the vacuum (15 mbar) overnight. The product was analyzed by PXRD and found to be Dabigatran etexilate bismesylate form Ms2-A
Yield: 11.25 g (99.9%)
Chemical purity (HPLC): 99.67 %
Residual solvent content (acetone and ethyl acetate): Below limit of detection

## Claims

1. Dabigatran Etexilate bismesylate salt.

2. The dabigatran etexilate bismesylate salt of claim 1, wherein the salt is crystalline.

3. The crystalline dabigatran etexilate bismesylate salt according to any preceding claim, **characterized by** data selected from: a PXRD pattern with peaks at 15.8, 20.4 and 24.3 degrees 2-theta ± 0.2 degrees 2-theta, a PXRD pattern substantially as depicted in Figure 1 (Ms2-A); and any combination thereof.

4. Dabigatran etexilate bismesylate salt according to any one of claims 1-3, **characterized by** a solid-state ¹³C NMR spectrum having characteristic peaks at 33.7, 41.8, 114.9, 120.0, 130.6 and 149.0 ppm, ± 0.2; a solid-state ¹³C NMR spectrum substantially as depicted in figure 4 or in figure 5; or by combinations thereof.

5. The crystalline dabigatran etexilate bismesylate salt according to any one of claims 1-4, **characterized by** an X-ray powder diffraction pattern having one, two or three additional peaks selected from the peaks at 19.1, 23.6, and 25.8 degrees 2-theta ± 0.2 degrees 2-theta.

6. The crystalline dabigatran etexilate bismesylate salt according to any one of claims 1-5, **characterized by** an X-ray powder diffraction pattern having peaks at 15.8, 19.1, 20.4, 23.6, 24.3 and 25.8 degrees two theta ± 0.2 degrees two theta.

7. The crystalline dabigatran etexilate bismesylate salt of any of claims 1-6, further **characterized by** the X-ray powder diffraction peaks listed in the following **Table 1:**
| **Angle 2-Theta ° ± 0.2** | **Intensity %** |
|---|---|
| 12,2 | 21 |
| 15.8 | 76 |
| 17.0 | 36 |
| 19.1 | 72 |
| 19.8 | 52 |
| 20.4 | 100 |
| 20.7 | 75 |
| 23.6 | 69 |
| 24.3 | 80 |
| 25.0 | 74 |
| 25.8 | 52 |
| 29.4 | 44 |

8. The crystalline dabigatran etexilate bismesylate salt of any one of claims 1-7, **characterized by** a DSC thermogram substantially as depicted in Figure 3.

9. The dabigatran etexilate bismesylate salt according to any one of claims 1-8, for use in the preparation of dabigatran etexilate, a salt thereof, especially the monomesylate salt, solid state forms thereof, and formulations thereof.

10. A process for preparing dabigatran etexilate, salts thereof, or solid state forms thereof, comprising preparing dabigatran etexilate bismesylate salt according to any of claims 1-8 and converting it to dabigatran etexilate, salts thereof or solid state forms thereof.

11. The process of claim 10, comprising:
a) basifying the dabigatran etexilate bismesylate salt according to any of claims 1-8; and optionally
b) reacting the product formed in step a) with an acid.

12. The process of claim 11, wherein dabigatran etexylate monomesylate salt is prepared and the acid is methanesulfonic acid.

13. The process of claim 12, wherein the molar ratio of dabigatran etexilate to methanesulfonic acid is from about 1: 0.95 to about 1: 0.99.

14. A pharmaceutical composition, especially an oral solid composition, such as a capsule or tablet, comprising dabigatran etexilate bismesylate salt according to any one of claims 1-8.

15. A pharmaceutical composition comprising the dabigatran etexilate bismesylate salt according to any one of claims 1-8 for use in treating or preventing blood clots in a patient suffering from, or susceptible to, the formation of blood clots.

## Patentansprüche

1. Dabigatranetexilat-Bismesylatsalz.

2. Dabigatranetexilat-Bismesylatsalz nach Anspruch 1, wobei das Salz kristallin ist.

3. Kristallines Dabigatranetexilat-Bismesylatsalz nach einem der vorhergehenden Ansprüche, **gekennzeichnet durch** Daten ausgewählt aus: einem PXRD-Muster mit Peaks bei 15,8, 20,4 und 24,3 Grad 2-Theta ± 0,2 Grad 2-Theta, einem im Wesentlichen wie in Fig. 1 gezeigten PXRD-Muster (Ms2-A); und einer beliebigen Kombination davon.

4. Dabigatranetexilat-Bismesylatsalz nach einem der Ansprüche 1-3, **gekennzeichnet durch** ein Festphasen-¹³C-NMR-Spektrum mit charakteristischen Peaks bei 33,7, 41,8, 114,9, 120,0, 130,6 und 149,0 ppm, ± 0,2; einem im Wesentlichen wie in Fig. 4 oder in Fig. 5 gezeigten Festphasen-¹³C-NMR-Spektrum; oder **durch** Kombinationen davon.

5. Kristallines Dabigatranetexilat-Bismesylatsalz nach einem der Ansprüche 1-4, **gekennzeichnet durch** ein Röntgenpulverbeugungsmuster mit einem, zwei oder drei zusätzlichen Peaks ausgewählt aus den Peaks bei 19,1, 23,6 und 25,8 Grad 2-Theta ± 0,2 Grad 2-Theta.

6. Kristallines Dabigatranetexilat-Bismesylatsalz nach einem der Ansprüche 1-5, **gekennzeichnet durch** ein Röntgenpulverbeugungsmuster mit Peaks bei 15,8, 19,1, 20,4, 23,6, 24,3 und 25,8 Grad 2-Theta ± 0,2 Grad 2-Theta.

7. Kristallines Dabigatranetexilat-Bismesylatsalz nach einem der Ansprüche 1-6, weiterhin **gekennzeichnet durch** die in der folgenden Tabelle 1 aufgeführten Röntgenpulverbeugungspeaks:
| **Winkel 2-Theta° ± 0,2** | **Intensität %** |
|---|---|
| 12,2 | 21 |
| 15,8 | 76 |
| 17,0 | 36 |
| 19,1 | 72 |
| 19,8 | 52 |
| 20,4 | 100 |
| 20,7 | 75 |
| 23,6 | 69 |
| 24,3 | 80 |
| 25,0 | 74 |
| 25,8 | 52 |
| 29,4 | 44 |

8. Kristallines Dabigatranetexilat-Bismesylatsalz nach einem der Ansprüche 1-7, **gekennzeichnet durch** ein im Wesentlichen wie in Fig. 3 gezeigtes DSC-Thermogramm.

9. Dabigatranetexilat-Bismesylatsalz nach einem der Ansprüche 1-8 zur Verwendung bei der Herstellung von Dabigatranetexilat, einem Salz davon, insbesondere dem Monomesylatsalz, Festphasenformen davon und Formulierungen davon.

10. Verfahren zur Herstellung von Dabigatranetexilat, Salzen davon oder Festphasenformen davon, bei dem man Dabigatranetexilat-Bismesylatsalz nach einem der Ansprüche 1-8 herstellt und dieses in Dabigatranetexilat, Salze davon oder Festphasenformen davon umwandelt.

11. Verfahren nach Anspruch 10, bei dem man:
a) das Dabigatranetexilat-Bismesylatsalz nach einem der Ansprüche 1-8 basisch stellt und gegebenenfalls
b) das in Schritt a) gebildete Produkt mit einer Säure umsetzt.

12. Verfahren nach Anspruch 11, bei dem man Dabigatranetexilat-Monomesylatsalz herstellt und es sich bei der Säure um Methansulfonsäure handelt.

13. Verfahren nach Anspruch 12, wobei das Molverhältnis von Dabigatranetexilat zu Methansulfonsäure etwa 1:0,95 bis etwa 1:0,99 beträgt.

14. Pharmazeutische Zusammensetzung, insbesondere eine orale feste Zusammensetzung wie eine Kapsel oder Tablette, welche Dabigatranetexilat-Bismesylatsalz nach einem der Ansprüche 1-8 umfasst.

15. Pharmazeutische Zusammensetzung, umfassend das Dabigatranetexilat-Bismesylatsalz nach einem der Ansprüche 1-8, zur Verwendung bei der Behandlung oder Prävention von Blutgerinnseln bei einem Patienten, der an der Bildung von Blutgerinnseln leidet oder gegenüber dieser empfindlich ist.

## Revendications

1. Sel de bismésylate d'étexilate de dabigatran.

2. Sel de bismésylate d'étexilate de dabigatran selon la revendication 1, le sel étant cristallin.

3. Sel de bismésylate d'étexilate de dabigatran cristallin selon une quelconque revendication précédente, **caractérisé par** des données choisies parmi : un diagramme de DRXP avec des pics à 15,8, 20,4 et 24,3 degrés 2-thêta ± 0,2 degré 2-thêta ; un diagramme de DRXP sensiblement comme représenté sur la figure 1 (Ms2-A) ; et toute combinaison de ceux-ci.

4. Sel de bismésylate d'étexilate de dabigatran selon l'une quelconque des revendications 1 à 3, **caractérisé par** un spectre de RMN ¹³C à l'état solide ayant des pics caractéristiques à 33,7, 41,8, 114,9, 120,0, 130,6 et 149,0 ppm ± 0,2 ; un spectre de RMN ¹³C à l'état solide sensiblement comme représenté sur la figure 4 ou sur la figure 5 ; ou toute combinaison de ceux-ci.

5. Sel de bismésylate d'étexilate de dabigatran cristallin selon l'une quelconque des revendications 1 à 4, **caractérisé par** un diagramme de diffraction des rayons X sur poudre ayant un, deux ou trois pics supplémentaires choisis parmi les pics à 19,1, 23,6 et 25,8 degrés 2-thêta ± 0,2 degré 2-thêta.

6. Sel de bismésylate d'étexilate de dabigatran cristallin selon l'une quelconque des revendications 1 à 5, **caractérisé par** un diagramme de diffraction des rayons X sur poudre ayant des pics à 15,8, 19,1, 20,4, 23,6, 24,3 et 25,8 degrés 2-thêta ± 0,2 degré 2-thêta.

7. Sel de bismésylate d'étexilate de dabigatran cristallin selon l'une quelconque des revendications 1 à 6, **caractérisé en outre par** les pics de diffraction des rayons X sur poudre répertoriés dans le tableau 1 suivant :
| **Angle 2-thêta (°) ± 0,2** | **Intensité (%)** |
|---|---|
| 12,2 | 21 |
| 15,8 | 76 |
| 17,0 | 36 |
| 19,1 | 72 |
| 19,8 | 52 |
| 20,4 | 100 |
| 20,7 | 75 |
| 23,6 | 69 |
| 24,3 | 80 |
| 25,0 | 74 |
| 25,8 | 52 |
| 29,4 | 44 |

8. Sel de bismésylate d'étexilate de dabigatran cristallin selon l'une quelconque des revendications 1 à 7, **caractérisé par** un thermogramme DSC sensiblement comme représenté sur la figure 3.

9. Sel de bismésylate d'étexilate de dabigatran selon l'une quelconque des revendications 1 à 8, à utiliser dans la préparation d'étexilate de dabigatran, d'un sel de celui-ci, en particulier du sel de monomésylate, de formes solides de celui-ci, et de formulations de celui-ci.

10. Procédé de préparation d'étexilate de dabigatran, de sels de celui-ci, ou de formes solides de celui-ci, comprenant la préparation du sel de bismésylate d'étexilate de dabigatran selon l'une quelconque des revendications 1 à 8 et sa transformation en étexilate de dabigatran, sels de celui-ci ou formes solides de celui-ci.

11. Procédé selon la revendication 10, comprenant :
a) la basification du sel de bismésylate d'étexilate de dabigatran selon l'une quelconque des revendications 1 à 8 ; et éventuellement
b) la réaction du produit formé à l'étape a) avec un acide.

12. Procédé selon la revendication 11, dans lequel le sel de monomésylate d'étexilate de dabigatran est préparé et l'acide est l'acide méthanesulfonique.

13. Procédé selon la revendication 12, dans lequel le rapport molaire de l'étexilate de dabigatran à l'acide méthanesulfonique est d'environ 1:0,95 à environ 1:0,99.

14. Composition pharmaceutique, en particulier une composition solide orale, telle qu'une capsule ou un comprimé, comprenant le sel de bismésylate d'étexilate de dabigatran selon l'une quelconque des revendications 1 à 8.

15. Composition pharmaceutique comprenant le sel de bismésylate d'étexilate de dabigatran selon l'une quelconque des revendications 1 à 8 à utiliser dans le traitement ou la prévention des caillots sanguins chez un patient souffrant de, ou sujet à, la formation de caillots sanguins.
